# EUROPEAN PATENT APPLICATION

(11) **EP 4 079 375 A1**
(43) Date of publication of application: **26.10.2022**
(21) Application number: 20881400.4
(22) Date of filing: 27.10.2020
(51) Int. Cl.: A61P 31/00, A61P 35/00, A61P 37/06, A61P 37/08, C12N 5/0783, A61K 35/17

(54) **METHOD FOR PRODUCING T CELLS HAVING CELL SURFACE MARKERS FOR CD45RA+ AND CCR7+**

(30) Priority: 28.10.2019 JP 2019195206
(71) Applicant: Daiichi Sankyo Company, Limited, Tokyo 103-8426 (JP); Keio University, Tokyo, 108-8345 (JP)
(72) Inventor: TOMISATO, Wataru, Tokyo 103-8426 (JP); YOSHIMURA, Akihiko, Tokyo 160-8582 (JP); KONDO, Taisuke, Tokyo 160-8582 (JP); ANDO, Makoto, Tokyo 160-8582 (JP)
(74) Representative: Vossius & Partner Patentanwälte Rechtsanwälte mbB
(86) International application number: PCT/JP2020/040163
(87) International publication number: WO 2021/085398

(57) **Abstract**

The present invention aims to solve a problem in T-cell transfer therapy and the like, which is T-cell exhaustion, and to provide a technique to enhance T cell activity. T cells having cell surface markers of CD45RA⁺ and CCR7⁺ can be produced by culturing activated T cells in the presence of (a) a conditioned medium derived from stromal cells or (b) CXCL12.

## Description

### TECHNICAL FIELD

The present invention relates to a method of producing T cells having cell surface markers of CD45RA⁺ and CCR7⁺. More particularly, the present invention enables the production of T cells having the cell surface markers of CD45RA⁺ and CCR7⁺ by culturing T cells with a conditioned medium derived from stromal cells or CXCL12.

### BACKGROUND ART

Adoptive T cell therapy is regarded as promising to treat cancer (Non Patent Literature 1). For example, it has been reported that patients with malignant melanoma achieved a complete response to tumor infiltrating lymphocyte (TIL) therapy (Non Patent Literatures 2 and 3). In this therapy, isolated TILs are restimulated with antigen presenting cells that present a tumor associated antigen (TAA) and then expanded, and the resulting TILs are administered to patients.

On the other hand, a new adoptive T cell therapy employing gene transfer techniques also exhibits dramatic therapeutic efficacy. The therapy is a method comprising transducing T cells derived from a patient with a T cell receptor (TCR) or chimeric antigen receptor (CAR) gene artificially designed to recognize a TAA or a therapeutic target molecule, expanding the transduced T cells in the presence of TCR stimulation, and returning the expanded T cells to the patient (Non Patent Literature 4).

However, these therapies have a problem in that transferred T cells often lose their functions, leading to a limited therapeutic efficacy (Non Patent Literatures 1 and 5). This is because such T cells undergo exhaustion and become dysfunctional in an expansion process or in a tumor microenvironment in which TILs have been present (Non Patent Literatures 6 and 7). Therefore, improvement of therapeutic efficacy of adoptive T cell therapy would be expected if the functions or retention of exhausted T cells can be restored.

Memory T cells have been divided into two sub-groups: effector memory T (T_{EM}) cells and central memory T (T_{CM}) cells (Non Patent Literature 8). Recently, stem cell memory T (T_{SCM}) cells, which are a new sub-group of memory T cells, have been identified. In humans, T_{SCM} is defined with the cell surface markers CD45RA⁺CD45RO⁻CCR7⁺CD62L⁺ (Non Patent Literatures 9 and 10). T_{SCM} is known to have a higher growth potential or a longer persistence in vivo and produce effector T cells having potent antitumor activity (Non Patent Literatures 11 and 12). In fact, it has been reported that elevated frequencies of T_{SCM}/early memory phenotypes in CAR-transduced T (CAR-T) cells in patients associate with sustained remission in CAR-T cell therapy (Non Patent Literatures 13 and 14).

The present inventors have already reported that induced stem cell memory-like T (iT_{SCM}) cells of which the cell surface markers are CD45RA⁺CD45RO⁻CCR7⁺CD62L⁺ can be obtained by co-culturing fully activated memory T cells or more differentiated memory T cells with feeder cells expressing a Notch ligand (Non Patent Literatures 12 and 15). This study has revealed that these functional iT_{SCM} cells have a superior anti-tumor ability than naive T cells or other subsets of memory T cells. The subsequent study by the present inventors has revealed that successful production of such T cells can be determined by confirming at least CD45RA⁺CCR7⁺ among CD45RA⁺CD45RO⁻CCR7⁺CD62L⁺. However, application of the method of producing iT_{SCM} cells to adoptive T cell therapy has a problem in that it is difficult to completely remove co-cultured feeder cells.

### CITATION LIST

### NON PATENT LITERATURE

NPL 1: June, C.H. (2007). Adoptive T cell therapy for cancer in the clinic. J Clin Invest 117, 1466-1476.
NPL 2: Wu, R., Forget, M.A., Chacon, J., Bernatchez, C., Haymaker, C., Chen, J.Q., Hwu, P., and Radvanyi, L.G. (2012). Adoptive T-cell therapy using autologous tumor-infiltrating lymphocytes for metastatic melanoma: current status and future outlook. Cancer J 18, 160-175.
NPL 3: Rosenberg, S.A. and Dudley, M.E. (2009). Adoptive cell therapy for the treatment of patients with metastatic melanoma. Curr Opin Immunol 21, 233-240.
NPL 4: Fesnak, A.D., June, C.H., and Levine, B.L. (2016). Engineered T cells: the promise and challenges of cancer immunotherapy. Nat Rev Cancer 16, 566-581.
NPL 5: Klebanoff, C.A., Acquavella, N., Yu, Z., and Restifo, N.P. (2011). Therapeutic cancer vaccines: are we there yet? Immunol Rev 239, 27-44.
NPL 6: Jiang, Y., Li, Y.,and Zhu, B. (2015). T-cell exhaustion in the tumor microenvironment. Cell Death Dis 6, e1792.
NPL 7: Gattinoni, L., Klebanoff, C.A., Palmer, D.C., Wrzesinski, C., Kerstann, K., Yu, Z., Finkelstein, S.E., Theoret, M.R., Rosenberg, S.A., and Restifo, N.P. (2005). Acquisition of full effector function in vitro paradoxically impairs the in vivo antitumor efficacy of adoptively transferred CD8+ T cells. J Clin Invest 115, 1616-1626.
NPL 8: Lanzavecchia, A. and Sallusto, F. (2005). Understanding the generation and function of memory T cell subsets. Curr Opin Immunol 17, 326-332.
NPL 9: Gattinoni, L. (2014). Memory T cells officially join the stem cell club. Immunity 41, 7-9.
NPL 10: Gattinoni, L., Speiser, D.E., Lichterfeld, M., and Bonini, C. (2017). T memory stem cells in health and disease. Nat Med 23, 18-27.
NPL 11: Gattinoni, L., Lugli, E., Ji, Y., Pos, Z., Paulos, C.M., Quigley, M.F., Almeida, J.R., Gostick, E., Yu, Z., Carpenito, C., et al. (2011). A human memory T cell subset with stem cell-like properties. Nat Med 17, 1290-1297.
NPL 12: Kondo, T., Morita, R., Okuzono, Y., Nakatsukasa, H., Sekiya, T., Chikuma, S., Shichita, T., Kanamori, M., Kubo, M., Koga, K., et al. (2017). Notch-mediated conversion of activated T cells into stem cell memory-like T cells for adoptive immunotherapy. Nat Commun 8, 15338.
NPL 13: Fraietta, J.A., Lacey, S.F., Orlando, E.J., Pruteanu-Malinici, I., Gohil, M., Lundh, S., Boesteanu, A.C., Wang, Y., O'Connor, R.S., Hwang, W.T., et al. (2018a). Determinants of response and resistance to CD19 chimeric antigen receptor (CAR) T cell therapy of chronic lymphocytic leukemia. Nat Med 24, 563-571.
NPL 14: Xu, Y., Zhang, M., Ramos, C.A., Durett, A., Liu, E., Dakhova, O., Liu, H., Creighton, C.J., Gee, A.P., Heslop, H.E., et al. (2014). Closely related T-memory stem cells correlate with in vivo expansion of CAR.CD19-T cells and are preserved by IL-7 and IL-15. Blood 123, 3750-3759.
NPL 15: Kondo, T., Imura, Y., Chikuma, S., Hibino, S., Omata-Mise, S., Ando, M., Akanuma, T., Iizuka, M., Sakai, R., Morita, R., and Yoshimura, A. (2018). Generation and application of human induced-stem cell memory T cells for adoptive immunotherapy. Cancer Sci 109, 2130-2140.

### SUMMARY OF INVENTION

### TECHNICAL PROBLEM

The present invention aims to solve T-cell exhaustion, a problem in T-cell transfer therapy, to enhance T cell activity. More specifically, the present invention aims to produce T cells having the cell surface markers of CD45RA⁺ and CCR7⁺.

### SOLUTION TO PROBLEM

The present inventors performed the intensive study to solve the problem and have found that T cells having the cell surface markers of CD45RA⁺ and CCR7⁺ can be produced by culturing activated T cells in the presence of a conditioned medium derived from stromal cells or CXCL12, leading to the completion of the present invention.

The present invention includes, but is not limited to, the following inventions [1] to [13]:
[1] A method of producing T cells having cell surface markers of CD45RA⁺ and CCR7⁺, wherein the method comprises a step of culturing T cells in the presence of a conditioned medium derived from stromal cells;
[2] The method according to [1], further comprising a step of activating T cells to obtain activated T cells;
[3] The method according to [1] or [2], wherein the stromal cell is any one or more selected from the group consisting of OP9 cell, TSt-4 cell, and these cells engineered to express a Notch ligand;
[4] The method according to any of [1] to [3], wherein the stromal cell is OP9-DLL1 cell;
[5] The method according to any of [1] to [4], further comprising a step of Notch signaling;
[6] The method according to [5], wherein the step of Notch signaling is performed using any one or more Notch ligands selected from the group consisting of DLL4, DLL1, JAG1, JAG2, and a recombinant thereof;
[7] The method according to [5] or [6], wherein the step of Notch signaling is performed using a recombinant DLL1;
[8] The method according to any of [5] to [7], wherein the step of Notch signaling is performed using forced expression of FOXM1 or NICD;
[9] The method according to any of [1] to [8], wherein the T cell having the cell surface markers of CD45RA⁺ and CCR7⁺ has cell surface markers of CD45RO⁻ and CD62L⁺;
[10] The method according to any of [1] to [9], the T cell is a CAR-T cell;
[11] A T cell produced by the method according to any of [1] to [10];
[12] A cellular medicine comprising the T cells according to [11]; and
[13] The cellular medicine according to [12], for the treatment of one or more selected from the group consisting of a cancer, an infection, an autoimmune disease, and an allergy.

Other aspects of the present invention include the following inventions [1A] to [14A]:
[1A] A method of producing T cells having the cell surface markers of CD45RA⁺ and CCR7⁺, wherein the method comprises a step of culturing source T cells in the presence of (a) a conditioned medium derived from stromal cells or (b) CXCL12;
[2A] The method according to [1A], further comprising a step of activating the source T cells before the culturing step;
[3A] The method according to [1A] or [2A], wherein the stromal cell is any one or more selected from the group consisting of OP9 cell, TSt-4 cell, and these cells engineered to express a Notch ligand;
[4A] The method according to any of [1A] to [3A], wherein the stromal cell is OP9-DLL1 cell;
[5A] The method according to any of [1A] to [4A], further comprising a step of activating Notch signal before or during the culturing step;
[6A] The method according to [5A], wherein the step of activating Notch signal is performed using any one or more Notch ligands selected from the group consisting of DLL4, DLL1, JAG1, JAG2, and a recombinant thereof;
[7A] The method according to [5A] or [6A], wherein the step of activating Notch signal is performed using a recombinant DLL1;
[8A] The method according to any of [5A] to [7A], wherein the step of activating Notch signal is performed using forced expression of FOXM1 or NICD;
[9A] The method according to any of [1A] to [8A], wherein the T cell having the cell surface markers of CD45RA⁺ and CCR7⁺ has the cell surface markers of CD45RO⁻ and CD62L⁺;
[10A] The method according to any of [1A] to [9A], wherein the source T cell is a CAR-T cell;
[11A] The method according to any of [1A] to [10A], wherein IGF-I is added in the step of culturing in the presence of CXCL12;
[12A] A T cell produced by the method according to any of [1A] to [11A];
[13A] A cellular medicine comprising the T cells according to [12A]; and
[14A] The cellular medicine according to [13A], for the treatment of one or more selected from the group consisting of a cancer, an infection, an autoimmune disease, and an allergy.

### ADVANTAGEOUS EFFECTS OF INVENTION

According to the production method of the present invention, T cells having cell surface markers of CD45RA⁺ and CCR7⁺ can be simply and efficiently obtained by culturing activated T cells in the presence of a conditioned medium (CM) derived from stromal cells or CXCL12.

Unlike conventional iT_{SCM} cells produced by co-culture with feeder cells, the present method does not require the step of removing feeder cells and thus is advantageous in the application to, for example, CAR-T therapy and adoptive T cell therapy.

### BRIEF DESCRIPTION OF DRAWINGS

[Fig. 1] Fig. 1 shows an experiment design scheme to cause overexpression of NICD in T cells.
[Fig. 2] Fig. 2 is a schematic of the construct of the NICD overexpression retroviral vector used to produce T cells forcedly expressing NICD.
[Fig. 3] Fig. 3 shows the expression of CD45RA and CCR7 in human CD8α⁺ T cells transduced with an empty vector (Empty) and human CD8α⁺ T cells forcedly expressing NICD (NICD) before and after the induction into stem cell memory-like T cells (left) and the number (%) of CD45RA⁺CCR7⁺ cells before and after the induction into stem cell memory-like T cells (right).
[Fig. 4] Fig. 4 is a schematic of the construct of the FOXM1 overexpression retroviral vector used to produce T cells forcedly expressing FOXM1.
[Fig. 5] Fig. 5 shows the expression of CD45RA, CCR7, and CD27 in T cells transduced with an empty vector (Empty) and T cells forcedly expressing FOXM1 gene (FOXM1ΔN) before and after the induction into stem cell memory-like T cells and the number (%) of CD45RA⁺CCR7⁺ and CD45RA⁺CCR7⁺CD27^{high} cells before and after the induction into stem cell memory-like T cells.
[Fig. 6] Fig. 6 shows the expression of CD45RA, CCR7, and CD27 in T cells transduced with an empty vector (Empty) and T cells forcedly expressing FOXM1 gene (FOXM1ΔN) before and after the induction into stem cell memory-like T cells and the number (%) of CD45RA⁺CCR7⁺ and CD45RA⁺CCR7⁺CD27^{high} cells before and after the induction into stem cell memory-like T cells.
[Fig. 7] Fig. 7 shows the expression of FOXM1 in T cells transduced with an empty vector or T cells forcedly expressing FOXM1 at day 0 (left) or day 11 (right), in which the expression of FOXM1 gene was normalized with the expression level of 18SrRNA.
[Fig. 8] Fig. 8 shows the expression of CD45RA and CCR7 in human CD8α⁺ T cells co-cultured with OP9-hDLL1 cells (on FC), T cells transduced with an empty vector and co-cultured with OP9-hDLL1 cells (Empty on FC), and FOXM1-forcedly expressing T cells cultured in the presence of a conditioned medium derived from OP9-hDLL1 cells (FOXM1ΔN in CM), when the FOXM1 inhibitor Thiostrepton or DMSO was added (n=3 in each group).
[Fig. 9] Fig. 9 shows the expression of CD45RA and CCR7 in human CD8α⁺ T cells co-cultured with OP9-hDLL1 cells (on FC), T cells transduced with an empty vector and co-cultured with OP9-hDLL1 cells (Empty on FC), and FOXM1-forcedly expressing T cells cultured in the presence of a conditioned medium derived from OP9-hDLL1 cells (FOXM1ΔN in CM), when the FOXM1 inhibitor Thiostrepton or DMSO was added (n=3 in each group)).
[Fig. 10] Fig. 10 represents graphs showing the expression of CD45RA and CCR7 (left: negative control, right: positive control).
[Fig. 11] Fig. 11 shows the results of electrophoresis for the determination of the gene expression of HPRT, CXCL12, SCF, IL-7, and FLT3L (Example 6).
[Fig. 12] Fig. 12 shows the quantification results of CXCL12 concentration (Example 6).
[Fig. 13] Fig. 13 is a graph showing the percentage of cells positive for both CD45RA and CCR7 (Example 7).
[Fig. 14] Fig. 14 is a graph showing the number of NALM6 cancer cells in blood taken from mice (Example 8).

### DESCRIPTION OF EMBODIMENTS

An aspect of the present invention is a method of producing T cells having the cell surface markers of CD45RA⁺ and CCR7⁺, comprising a step of culturing T cells in the presence of a conditioned medium derived from stromal cells. Another aspect of the present invention is a method of producing T cells having the cell surface markers of CD45RA⁺ and CCR7⁺, comprising a step of culturing T cells in the presence of CXCL12. According to the methods of the aspects, T cells having the cell surface markers of CD45RA⁺ and CCR7⁺ can be obtained without co-culture with feeder cells.

The methods of the present invention are not particularly limited as long as the methods comprise a step of culturing T cells in the presence of a conditioned medium derived from stromal cells or CXCL12, and other steps of the methods may be performed by combining known steps. The present invention may comprise, for example, the following:
(1) a step of activating T cells (activation step);
(2) a step of transferring a gene into T cells (gene transfer step);
(3) a step of activating Notch signal (Notch signaling step); and/or
(4) a step of harvesting, separating, and/or purifying stem cell memory-like T cells having the cell surface markers of CD45RA⁺ and CCR7⁺. Unless otherwise specified below, the order of these steps shall be appropriately selected by those skilled in the art. Step (3) may be performed simultaneously with step (1) or (2).

Generally, a T cell means a cell having TCR on the surface. In this specification, the "T cell" may be, in addition to T cell itself, a cell population comprising other types of cells, for example, a cell population comprising 60% or more, 70% or more, 80% or more, 90% or more, or 95% or more T cells. T cells include those in all differentiation stages and may be engineered to have an antigen or a marker on the surface.

In this specification, the "stem cell memory-like T cell" means a T cell having the cell surface markers of CD45RA⁺ and CCR7⁺. In a preferred aspect, stem cell memory-like T cells have cell surface markers of CD45RO⁻ and CD62L⁺ in addition to CD45RA⁺ and CCR7⁺. Stem cell memory-like T cells may include, for example, known cells also known as T_{SCM} cells or iT_{SCM} cells.

In this specification, the "activated T cell" means a T cell having cell surface markers of CD4⁺CCR7⁻CD45RA⁻ or CD8⁺CCR7⁻CD45RA⁻. In a preferred aspect, an activated T cell has cell surface markers of CD8⁺CCR7⁻CD45RA⁻. In an aspect, source T cells in the step of culturing T cells in the presence of a conditioned medium derived from stromal cells or CXCL12 may be activated T cells. In this case, the source T cells are synonymous with activated T cells.

Activated T cells may be obtained or prepared by any methods known to those skilled in the art. For example, activated T cells may be obtained by using, as a source, commercially available T cell populations, T cell populations taken from human (e.g., a healthy donor, a donor suffering from a particular disease, and a patient itself), or desired T cells separated and concentrated from these T cell populations. For example, activated T cells can be prepared by stimulating commercially available human peripheral blood mononuclear cells (Precision Bioservices, Inc.) with anti-CD3/CD28 antibodies.

Activated T cells that can be used in the present invention may be, in addition to T cells obtained from the methods as described above, T cells that are prepared by inducing differentiation of reprogrammed undifferentiated cells, such as ES cells and induced pluripotent stem cells (iPS cells), or by inducing differentiation of immature T cells such as naive T cells. Activated T cells that can be used in the present invention may be also activated T cells modified to express a particular T cell receptor (TCR) or chimeric antigen receptor (CAR) through a gene transfer step or activated T cells engineered to express a Notch-associated gene by activating Notch signal.

Stromal cells, also referred to as interstitial cells, are cells that provide an environment in which stem cells or rejuvenated cells maintain their survival and differentiate. When used as feeder cells, stromal cells are thought to give contact stimulation of cells and provide or produce nutritional factors. In this specification, the "stromal cell" means a stromal cell or a cell modified therefrom. Stromal cells include, for example, OP9 cells derived from bone marrow, TSt-4 cells, and these cells engineered to express a Notch ligand (e.g., OP9-DLL1 cells and TSt-4-DLL1 cells). Stromal cells are preferably OP9 cells or OP9 cells engineered to express a Notch ligand, more preferably, OP9-hDLL1 cells.

The Notch ligand that is to be expressed on a stromal cell includes, for example, DLL1, DLL4, JAG1, JAG2, and a combination thereof. The Notch ligand may be full, partial, or modified and is preferably DLL1, more preferably human DLL1 (hDLL1).

It has been known that OP9 cells that are engineered to express a Notch ligand can be used to differentiate pluripotent stem cells into cells of various tissues. For example, mouse-derived OP9-DLL1 cells are often used in vitro to cause development of immune cells and to induce differentiation of iPS cells into T cells. Culturing OP9-DLL1 cells in the presence of a conditioned medium has been reported to allow differentiation of immature T cells into mature T cells. However, it has not been known that a conditioned medium derived from stromal cells or CXCL12 can be used to induce mature T cells into stem cell memory-like T cells.

In this specification, the "conditioned medium derived from stromal cells" can be prepared by culturing stromal cells in a medium and collecting the conditioned medium. For example, it can be obtained by culturing stromal cells in αMEM (Gibco) supplemented with fetal bovine serum (20%, FBS), a penicillin-streptomycin solution (1%, ThermoFisher), and the like at 37°C in 5 vol.% CO₂ for one to two days, harvesting the conditioned medium, and then filtering it through a 0.45 micron filter. The medium during the culture can be replaced appropriately, for example, when the cells reached 100% confluence. A concentration of stromal cells contained in a culture medium is not particularly limited, but is preferably 1 to 2 × 10⁵ cells/10 ml/10 cm dish.

"CXCL12" is a CXC motif chemokine. CXCL12 and its receptor CXCR4 are involved in the development of B cells and myeloid cells and homing of hematopoietic stem cells to bone marrow. Janas et al., reported that OP9-DLL1 cells produce CXCL12 and that CXCL12/CXCR4 signaling contributes to proliferation and differentiation of progenitor T cells (Michelle L. Janas et al. J Exp Med. 2010 Jan 18; 207(1): 247-261).

The "step of culturing T cells in the presence of a conditioned medium derived from stromal cells" in this specification is not particularly limited as long as a conditioned medium derived from stromal cells is used in the step. The step can be performed by any culture method known to those skilled in the art. The "step of culturing T cells in the presence of CXCL12" in this specification is not particularly limited as long as CXCL12 is used in the step. The step can be performed by any culture method known to those skilled in the art (hereinafter also simply referred to as a culturing step instead of the step of culturing T cells in the presence of a conditioned medium derived from stromal cells and the step of culturing T cells in the presence of CXCL12).

A medium and an additive used in the culturing step are not particularly limited, and any medium and additive generally known to be suitable for cell attachment and cell induction may be used by coating them on or adding them to a commercially available culture vessel. Examples of the medium include αMEM (Gibco). Examples of the additive include various cytokines such as human IL-7 (PeproTech).

The culturing methods or conditions in this step are not particularly limited as long as they can produce stem cell memory-like T cells from T cells (especially, activated T cells). Any culturing methods or conditions known to those skilled in the art may be used. For example, activated T cells are seeded in a medium supplemented with additive agents, and a conditioned medium derived from stromal cells or CXCL12 is added to the medium, followed by culture at a temperature of about 37°C for 9 to 11 days, more preferably 10 to 11 days. The conditioned medium may be replaced every 2 to 5 days, preferably every 2 to 4 days. For example, T cells are cultured in the presence of a conditioned medium derived from OP9-hDLL1 (1 to 10 ml) or CXCL12 at a temperature of 37°C for 11 days, and the conditioned medium is replaced every 2 to 4 days.

In an aspect, the culturing step requires no feeder cells. The phrase "requires no feeder cells" as used herein means that the culturing step is performed under a culture condition substantially free from feeder cells, especially a condition other than co-culture with feeder cells.

The present invention may include a step of activating Notch signal (also referred to as Notch signaling step). Activation of Notch signal will initiate or mediate signaling associated with a Notch ligand. Activation of Notch signal is not particularly limited, but is preferably performed using at least one selected from the group consisting of a recombinant DLL1, forced expression of FOXM1, forced expression of NICD, and a combination thereof.

"Activation of Notch signal" is known to play an important role in cell fate determination, such as proliferation or differentiation of cells, in various processes of building tissues in a development process. There are four Notch receptors: NOTCH1, NOTCH2, NOTCH3, and NOTCH4. Notch receptors are cell surface receptors that transduce a short range signal by interacting with a transmembrane ligand such as Delta (referred to as Delta-like in human) or Serrate (referred to as Jagged in human) on an adjacent cell. In other words, members of Delta-like (DLL1, DLL3, DLL4) and Jagged (JAG1, JAG2) family present on a cell that transmits a signal serve as ligands to Notch receptors.

The "Notch ligand" in the activation of Notch signal includes DLL4, DLL1, JAG1, JAG2, and a combination thereof, as described above for the Notch ligands regarding a stromal cell. The Notch ligand may be full, partial, or modified and is preferably DLL1, more preferably hDLL1.

The type of "Notch receptor" is not particularly limited, but is preferably NOTCH1, NOTCH2, or a combination thereof.

The activation of Notch signal can be performed on a solid material (substrate) to which a Notch ligand peptide is immobilized. For example, the Notch ligand peptide is a soluble peptide of various Notch ligands and may be coupled to/immobilized on a solid material such as a polystyrene plate or beads. When a "recombinant DLL1" is used, for example, human Fc-DLL1 (Adipogen Life Sciences) immobilized on a polystyrene plate is used. In this case, the activation of Notch signal can be performed simultaneously with the culturing step. The activation of Notch signal performed simultaneously with the culturing step is advantageous in that it eliminates the need for previously producing T cells that have been engineered to forcedly express a gene involved in the activation of Notch signal.

The activation of Notch signal may be also performed by using T cells that have been engineered to forcedly express a gene involved in Notch signal. In other words, the activation of Notch signal includes forced expression in activated T cells of a gene involved in Notch signaling. The type of genes is not particularly limited, and examples include FOXM1 and NICD. Such genes may have a complete, partial, or modified sequence and may have N-terminus-deleted FOXM1 (FOXM1ΔN), for example. The forced expression of genes may be performed according to any methods known to those skilled in the art.

"NICD" means a Notch intracellular domain and is known to be cleaved and released upon the binding of a Notch ligand, followed by nuclear import of NICD to activate a target gene of Notch.

"FOXM1" (forkhead box protein M1) is known to regulate genes associated with cell cycle progression through the G2/M checkpoint and other cell cycle-associated genes. FOXM1 is also known to regulate, in addition to cell cycle, stemness, mitochondrial functions, and redox networks in different tumors. FOXM1 has not been known to be involved in Notch signaling, but in the study described in Experiments 3 and 4 below, the present inventors found for the first time that FOXM1 is a main downstream gene in Notch signaling and is likely to positively regulate induction of T_{SCM}-like phenotype.

In the present invention, T cells may be cultured in the presence of CXCL12 and IGF-I. "IGF-I" is a single stranded polypeptide with a molecular structure similar to that of insulin. IGF-I is produced in the liver in a growth hormone-dependent manner and is involved in proliferation of various cells and bone formation. IGF-I has been also reported to play an important role in differentiation of progenitor T cells (O Kecha et al. Endocrinology. 2000 Mar;141(3):1209-17).

"SCF", "IL-7", and "FLT3L" are all a cytokine or a growth factor that is involved in development and differentiation of lymphocytes including progenitor T cells (P J Morrissey et al. Cell Immunol. 1994 Aug;157(1):118-31., T A Moore and A Zlotnik J Immunol. 1997 May 1;158(9):4187-92). In this specification, hypoxanthine phosphoribosyltransferase (HPRT) is a housekeeping gene and is used as a control.

In this specification, the "step of activating T cells" is typically a step performed before the culturing step and can include all steps to be performed to obtain activated T cells. This step includes, for example, a step of obtaining activated T cells, such as induction of differentiation with anti-CD3/CD28 antibody bead, B cells transformed with EB virus, or CAR or TCR gene transduction, or a gene transfer step of transferring a gene into T cells, and a combination thereof. These steps may be performed in the order suitable to obtain activated T cells of interest.

In this specification, the "gene transfer step of transferring a gene into T cells" means a step to obtain genetically modified T cells. The gene transfer can be performed on T cells at all stages of development, in which genes and gene transfer methods to be used are not particularly limited. One or more genes can be transferred, depending on properties or functions that should be imparted to stem cell memory-like T cells of interest. For example, activated T cells obtained from the gene transfer step are CAR-T cells. The genetic information transduced is maintained after the culturing step of this aspect, and thus activated T cells having a desired genetic information can be used to obtain stem cell memory-like T cells that can be used in a cell therapy of interest. T cells may be autologous or allogeneic.

Examples of the gene transfer include one or more of (i) deletion or decrease of expression of B2M, TAP1, TAP2, tapasin, NLRC5, LAG3, TIM3, RFXANK, CITTA, RFX5, or RFXAP; (ii) expression of or increased expression of PD?1, SOCS1/3, HLA-E, HLA-G, HACD16, 41BBL, CD3, CD4, CD8, CD47, CD137, CD80, PDL1, A2AR, activated STAT3, CAR, TCR, or an inducible surface receptor to a bispecific or multispecific engager. The gene transfer is particularly preferably expression of CAR.

Chimeric antigen receptor (CAR) is composed of an intracellular domain that is present inside the cell membrane of a T cell, an extracellular domain that is present outside the cell membrane, and a spacer and a transmembrane domain connecting these domains. The intracellular domain has a CD3ζ chain linked to a co-stimulatory domain such as CD28 or 4-1BB. The co-stimulatory domain enhances cell proliferation of CAR-T cells and serves to preserve antiapoptotic functions originally provided by endogenous T cells. The CD3ζ chain causes signal activation and amplification of CAR-T. The extracellular domain, single-chain variable fragment (scFv), binds to a tumor antigen via the targeting elements. In the present invention, a CAR construct may be any construct known in the art. An antigen-binding domain in CAR is, for example, CD19 or CD20 and is preferably CD19.

Genetic engineering for obtaining CAR-T cells may be achieved by any method known to those skilled in the art and is easily modified for applying to the methods of the present invention as appropriate. For example, a CAR construct is incorporated into an expression vector. Various expression vectors are known in the art, and any of such vectors may be used. In a preferred embodiment, the vector is a retroviral or lentiviral vector. Electroporation or transfection via lipofection may be also used.

In the implementation of the present invention, unless otherwise specified, those who carry out the present invention can refer to standard books in the art for methods of molecular biology and genetic engineering such as DNA recombination techniques, common methods of cell biology, and conventional techniques. Examples of such books include the following:
"Molecular Cloning: A Laboratory Manual" (Sambrook & Russell, Cold Spring Harbor Laboratory Press, Third edition, 2001);
"Current Protocols in Molecular biology" (edited by Ausubel et al., John Wiley & Sons, 1987);
A series of "Methods in Enzymology" (Academic Press);
"PCR Protocols: Methods in Molecular Biology" (edited by Bartlett & Striling, Humana Press, 2003);
"Animal Cell Culture: A Practical Approach" (edited by Masters, Oxford University Press, Third edition, 2000);
"Antibodies: A Laboratory Manual" (edited by Harlow et al. & Lane, Cold Spring Harbor Laboratory Press, 1987); and
"Optimization of Methods for the Genetic Modification of Human T Cells" (Mahmood Y. Bilal, Aldo Vacaflores, and Jon C.D. Houtman; Immunol Cell Biol. 2015 Nov; 93(10): 896-908),
which are incorporated herein by reference.

Reagents and kits for cell culture and cell biological experiments as referenced herein are available from vendors such as Sigma-Aldrich Co. LLC, Invitrogen, Clontech, R&D Systems, Inc., BD Bioscience, Lonza, eBioscience, Inc., Thermo Fisher Scientific Inc., PeproTech, Inc., Bio Legend, Inc., Gibco, and NACALAI TESQUE, INC.

Stem cell memory-like T cells obtained from the method of this aspect may subsequently be harvested, separated, and purified by any known methods. The stem cell memory-like T cells can be identified by determining the expression of the cell surface markers of CD45RA⁺ and CCR7⁺. The expression of markers can be determined by any techniques, especially known cellular and histological techniques and molecular biological techniques including immunostaining with an antibody, reverse transcriptase-mediated polymerase chain reaction (RT-PCR), and hybridization analysis.

A method of purifying stem cell memory-like T cells may be any known methods of separating and purifying the cells. Specific examples of the methods include techniques utilizing an antigen-antibody reaction, such as flow cytometry, magnetic beads, and panning; and cell fractionation via density gradient centrifugation with sucrose or a carrier such as Percoll.

Those who carry out the present invention can refer to standard books in the art for common methods of preparation, subculture, and preservation of various cells and cell biological experiments used in the present invention. Examples of these include "Cell Culture Protocols (in Japanese) " (edited by Yukio Nakamura; YODOSHA CO., LTD.) which is incorporated herein by reference. Reagents and kits for cell culture and experiments of development and cell biology as referenced herein are available from vendors such as Invitrogen and Sigma-Aldrich Co. LLC.

In another aspect, the present invention relates to CAR-T cells having the cell surface markers of CD45RA⁺ and CCR7⁺, in which the CAR-T cells are preferably stem cell memory-like CAR-T cells.

The "CAR-T cells " in this aspect may be autologous or allogeneic CAR-T cells, as long as they have the phenotype as described above. The CAR-T cells may be obtained or prepared by any methods known to those skilled in the art and can be obtained by the method of producing stem cell memory-like T cells as described above. CAR-T cells of this aspect can be used for disease-specific adoptive T cell therapies. Examples of cell surface markers that can act as a ligand to an antigenic domain in CAR include cell surface markers associated with cancers, viral, bacterial, and parasitic infections, autoimmune diseases, and allergies.

For example, CAR-T cells can be transplanted or administered for the treatment of one or more diseases selected from the group consisting of various cancers, infections, autoimmune diseases, and allergies. Examples of the cancers include hematopoietic cell malignancies and solid cancers. Examples of the hematopoietic cell malignancies include acute lymphoblastic leukemia (ALL), acute myeloid leukemia (AML), chronic myelogenous leukemia (CML), chronic eosinophilic leukemia (CEL), myelodysplastic syndrome (MDS), non-Hodgkin's lymphoma (NHL), and multiple myeloma (MM). Examples of the solid cancers include biliary tract cancer, bladder cancer, bone and soft tissue carcinomas, brain tumor, breast cancer, cervical cancer, colon cancer, colorectal adenocarcinoma, colorectal cancer, desmoid tumor, embryonal cancer, endometrial cancer, esophageal cancer, gastric cancer, gastric adenocarcinoma, glioblastoma multiforme, gynecologic neoplasm, head and neck squamous cell carcinoma, liver cancer, lung cancer, malignant melanoma, osteosarcoma, ovary cancer, pancreatic cancer, pancreatic ductal adenocarcinoma, primary astrocytic tumor, primary thyroid cancer, prostate cancer, kidney cancer, renal cell carcinoma, rhabdomyosarcoma, skin cancer, soft tissue sarcoma, testicular germ cell tumor, urothelial carcinoma, uterine sarcoma, and uterine cancer.

CAR-T cells can be, for example, expanded, washed, and concentrated with phosphate-buffered physiological saline and then formulated with sterile physiological saline supplemented with human albumin (e.g., 5% Albumex(R) 20). CAR-T cells can be administered at an appropriate cell dose determined by those skilled in the art. In an aspect, CAR-T cells are administered as live cells at a dose of 1.2 x 10⁶ to 6 x 10⁸ or more cells. The maximum number of reinjected or transplanted T cells can be determined in a clinical trial performed by those skilled in the art.

In further another aspect, the present invention relates to an agent for cell therapy comprising stem cell memory-like T cells obtained from the production methods as described above or CAR-T cells having the cell surface markers of CD45RA⁺ and CCR7⁺. For example, a pharmaceutical composition comprising stem cell memory-like T cells or CAR-T cells as a substantially active ingredient can be administered. More specifically, it can be administered for the treatment of human immune diseases or cancers.

### EXAMPLES

The present invention will be described more specifically in Examples below. The present invention is not limited to the following specific examples. In this specification, unless otherwise specified, a range of numerical values includes its end points, and concentration and the like are by weight.

### [Antibodies and Reagents]

Anti-mouse Notch 1 antibody (HMN1-12), anti-mouse Notch2 antibody (HMN2-35), anti-mouse Notch3 antibody (HMN3-133), anti-human CD8a antibody (HIT8a), anti-human CD45RA antibody (HI100), anti-human CD45RO antibody (UCHL1), anti-human CCR7 antibody (G043H7), anti-human CD62L antibody (DREG-56), and anti-human CD27 antibody (M-T271) were purchased from eBioscience, Inc. (San Diego, CA, USA) or BioLegend, Inc. (San Diego, CA, USA) and used. Recombinant human DLL1 protein can be purchased from Adipogen Life Sciences (San Diego, CA, USA).

### [Statistical Analysis]

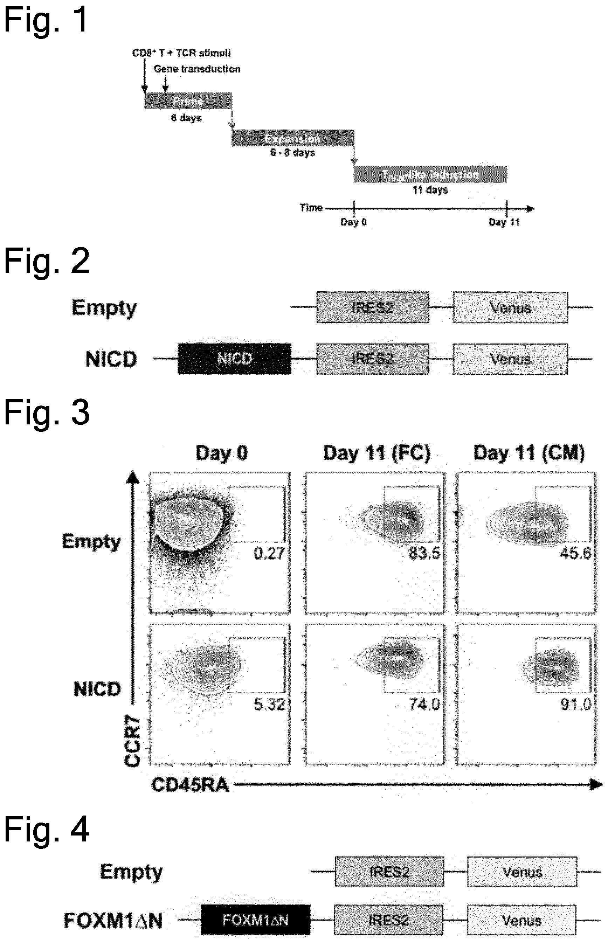

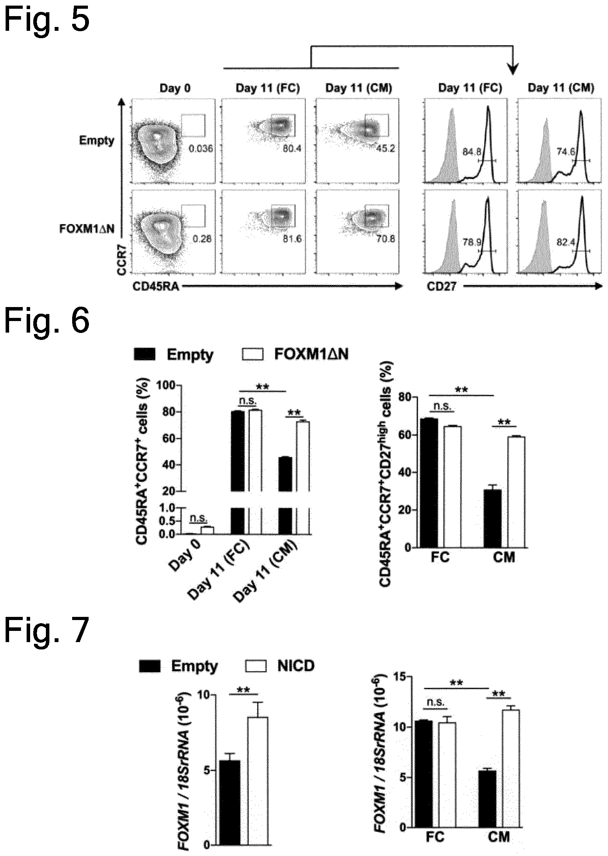

In each of the Figures, "^{∗∗}" represents p<0.01; "^{∗}" represents p<0.05; "n.s." represents not significant; and an error bar represents standard error of mean (s.e.m.). In Figs. 7, 8 and 9, one-way analysis of variance (one-way ANOVA) was used to perform statistical analysis. In Figs. 4 and 6, two-way analysis of variance (two-way ANOVA) was used to perform statistical analysis. Unless otherwise specified, each data is representative of at least two independent experiments from two different healthy donors.

### [Flow cytometric analysis and cell sorting]

To quantify the expression of cell surface markers, cells were collected, resuspended in phosphate-buffered physiological saline (PBS) containing 0.5% bovine serum albumin (BSA) and 2 mM ethylenediaminetetraacetic acid (EDTA), and surface-stained in the presence of an Fc-blocking antibody (2.4G2). The antibodies were purchased from BioLegend, Inc., eBioscience, Inc., R&D Systems, Inc., Beckman Coulter, Inc., BD Biosciences, and Cell Signaling Technology, Inc. Each of the antibodies was diluted at 1:100, and the diluted antibodies were used to perform intracellular staining. The resulting cells were analyzed by flow cytometry on a FACS Canto II cytometer (BD Biosciences, San Jose, CA, USA). The data were analyzed with FlowJo software (Tree Star, Inc., Ashland, OR, USA). Mouse and human T cells were sorted on a FACS Aria II or FACS Aria III cell sorter (BD Biosciences), or an SH800 cell sorter (Sony Biotechnology Inc., Tokyo, Japan).

### Example 1: Production of stem cell memory-like T cells in the presence of a conditioned medium derived from OP9-hDLL1

### 1-1. Preparation of a conditioned medium derived from OP9-hDLL1

A conditioned medium derived from OP9-hDLL1 cells (obtained from RIKEN) (OP9-hDLL1 CM) was prepared. OP9-hDLL1 cells were seeded onto a 10 cm dish for cell culture (Falcon, 353003) at 1.5 × 10⁵ cells/dish and grown in αMEM medium (Gibco, 10 mL) supplemented with fetal bovine serum (20%, FBS, Sigma-Aldrich Co. LLC) and a penicillin-streptomycin solution (1%, Thermo Fisher Scientific Inc.) The cells were grown under the conditions at 37°C in 5 vol.% CO₂. When the cells reached 100% confluence, the medium was harvested and filtered through a sterile filter (pore size: 0.45 µm) to obtain a conditioned medium, which was stored at 4°C.

### 1-2. Preparation of activated T cells (activated CD8α⁺ T cells)

[Step of sorting source T cells] Human peripheral blood mononuclear cells (PBMCs) were prepared from peripheral blood donated by three healthy donors who were Epstein-Barr virus (EBV) seropositive (VCA-IgG [+], EBNA [+]) by density gradient centrifugation. All cells except CD8α⁺ T cells were removed from the PBMCs using a human CD8α⁺ T cell isolation kit (#130-096-495, Miltenyi Biotec) to prepare human CD8α⁺ T cells.

[Priming step] The obtained human CD8α⁺ T cells were activated with microbeads to which an anti-CD3 antibody and an anti-CD28 antibody had been conjugated (CD3/CD28 Dynabeads, VERITAS Corporation), at a ratio of T cells to beads of 1:1 for 6 days.

[Expansion step] Six days after the priming step, activated CD8α⁺ T cells were subjected to TCR stimulation and sorted for CD8a⁺CD45RA⁻ by fluorescence-activated cell sorting (FACS) to obtain activated CD8α⁺ T cells.

### 1-3. Preparation of stem cell memory-like T cells in the presence of a conditioned medium derived from OP9-hDLL1

The obtained activated T cells were seeded onto a plate and cultured in OP9-hDLL1 CM (0.4 mL) supplemented with human IL-7 (PeproTech, Inc., 10 ng/mL) for 11 days to obtain stem cell memory-like T cells (T_{SCM}) (CM).

As a control, the activated T cells were co-cultured with OP9-hDLL1 cells (2 × 10³, feeder cells) in the presence of human IL-7 (PeproTech, Inc., 10 ng/mL) for 11 days to obtain induced stem cell memory-like T cells (iT_{SCM}) (FC).

### 1-4. Analysis of surface marker expression

The stem cell memory-like T cells obtained from the culture in the presence of the conditioned medium derived from OP9-hDLL1 were examined for the expression of surface antigen markers by the flow cytometric analysis described above, and it was confirmed that the cells expressed CD45RA⁺ and CCR7⁺ (Table 1).

### Example 2: Production of stem cell memory-like T cells in the presence of a conditioned medium derived from OP9 or TSt-4 cells

### 2-1. Preparation of conditioned media derived from OP9 and TSt-4 cells (OP9 CM and TSt-4 CM)

Conditioned media (OP9 CM and TSt-4 CM) derived from OP9 cells (obtained from RIKEN) and TSt-4 cells (obtained from RIKEN) were prepared. OP9 cells at 1.5 x 10⁵ cells/dish and TSt-4 cells at 1.5 × 10⁵ cells/dish were individually seeded onto a 10 cm dish for cell culture (Falcon 353003) and grown in αMEM medium (Gibco, 10 mL) supplemented with FBS (20%, Sigma-Aldrich Co. LLC) and a penicillin-streptomycin solution (1%, Thermo Fisher Scientific Inc.). The cells were grown under the conditions at 37°C in 5 vol.% CO₂. When the cells reached 100% confluence, the media were harvested and filtered through a sterile filter (pore size: 0.45 µm) to obtain conditioned media (OP9 CM and TSt-4 CM), which were stored at 4°C.

### 2-2. Preparation of activated T cells (activated CD8α⁺ T cells)

[Step of sorting source T cells] All cells except CD8α⁺ T cells were removed from PBMCs using a human CD8α⁺ T cell isolation kit (#130-096-495, Miltenyi Biotec) to prepare human CD8α⁺ T cells.

[Priming step] The obtained human CD8α⁺ T cells were activated with microbeads to which an anti-CD3 antibody and an anti-CD28 antibody had been conjugated (CD3/28 Dynabeads, VERITAS Corporation) at a ratio of T cells to beads of 1:1 for 6 days.

[Expansion step] Six days after the priming step, the activated CD8α⁺ T cells were subjected to TCR stimulation and sorted for CD8α⁺CD45RA⁻ by FACS to obtain activated CD8α⁺ T cells.

### 2-3. Preparation of stem cell memory-like T cells in the presence of OP9 CM or TSt-4 CM

The obtained activated T cells were seeded onto a plate and cultured in OP9 CM (0.4 mL) or TSt-4 CM (0.4 mL) supplemented with human IL-7 (PeproTech, Inc., 10 ng/mL) for 11 days to obtain stem cell memory-like T cells.

### 2-4. Analysis of surface marker expression

The stem cell memory-like T cells obtained from the culture in the presence of a conditioned medium derived from stromal cells (OP9 CM or TSt-4 CM) were examined for the expression of surface antigen markers by the flow cytometric analysis described above, and it was confirmed that the cells expressed CD45RA⁺ and CCR7⁺ (Table 1).

### Example 3: Production of stem cell memory-like T cells using OP9-hDLL1-derived conditioned medium/Notch signal activation (forced expression of NICD)

### 3-1. Preparation of activated T cells with forced expression of NICD (NICD-forcedly expressing activated CD8α⁺ T cells)

According to an experiment design scheme to cause the overexpression of Notch intracellular domain (NICD, SEQ ID NO: 1) in activated T cells as shown in Fig. 1, an NICD overexpression retroviral vector as shown in Fig. 2 was produced (pMEI-5 DNA, Takara Bio Inc.). The produced retroviral vector was used to produce NICD-forcedly expressing activated T cells.

Specifically, human CD8α⁺ T cells were activated in a similar manner to the Priming step described in Example 1. Twenty-four hours after the activation, the activated T cells were transduced with the NICD overexpression retroviral vector to achieve forced expression of NICD. Six days after the activation of T cells, the cells were sorted for NICD-forcedly expressing T cells as Venus⁺ cells, and the sorted cells were re-activated with CD3/28 microbeads.

The resulting cells were sorted for CD8α⁺CD45RA⁻ by FACS according to a method similar to the expansion step described in Example 1 to obtain NICD-forcedly expressing activated CD8α⁺ T cells.

### 3-2. Culture in the presence of a conditioned medium derived from OP9-hDLL1

The obtained NICD-forcedly expressing activated CD8α⁺ T cells were cultured in OP9-hDLL1 CM according to a method similar to that described in Example 1 to obtain stem cell memory-like T cells forcedly expressing NICD.

As a control, activated CD8α⁺ T cells that had been produced by transducing them with an empty vector were cultured under the same conditions to prepare stem cell memory-like T cells.

### 3-3. Analysis of surface marker expression

The obtained stem cell memory-like T cells forcedly expressing NICD (NICD) and stem cell memory-like T cells produced by transducing them with an empty vector (Empty) were examined for reactivity to antibodies specific to the markers described in Example 1. In addition, iT_{SCM} cells that were obtained by co-culturing activated T cells that had been transduced with NICD or an empty vector with OP9-hDLL1 cells were analyzed for the expression as a positive control.

The results shown in Fig. 3 and Table 1 confirm that the combination of Notch signal activation resulting from the forced expression of NICD and culture in the presence of OP9-hDLL1-derived conditioned medium (CM) allowed the expression of surface antigen markers of CD45RA⁺ and CCR7⁺ as in co-culturing with OP9-hDLL1 cells (FC).

### Example 4: Production of stem cell memory-like T cells using OP9-hDLL1-derived conditioned medium/Notch signal activation (forced expression of FOXM1)

### 4-1. Preparation of activated T cells forcedly expressing FOXM1 (FOXM1ΔN-forcedly expressing activated CD8α⁺ T cells)

Instead of the NICD overexpression retroviral vector, a FOXM1ΔN (SEQ ID NO: 2) overexpression retroviral vector as shown in Fig. 4 was used to prepare FOXM1ΔN-forcedly expressing activated CD8α⁺ T cells, which are T cells forcedly expressing N-terminus-deleted FOXM1, according to a method similar to the method of preparing NICD-forcedly expressing activated CD8α⁺ T cells described in Example 3.

### 4-2. Culture in the presence of a conditioned medium derived from OP9-hDLL1

The obtained FOXM1ΔN-forcedlyexpressing activated CD8α⁺ T cells were cultured in the OP9-hDLL1 CM according to a method similar to that described in Example 1 to obtain FOXM1ΔN-forcedly expressing stem cell memory-like T cells. The obtained cells were transduced with an empty vector as in Example 3 to prepare stem cell memory-like T cells as a control.

### 4-3. Analysis of surface marker expression

The obtained FOXM1ΔN-forcedly expressing stem cell memory-like T cells (FOXM1ΔN) and stem cell memory-like T cells (Empty) were examined for reactivity to antibodies specific to the markers described in Example 1 and to an antibody specific to an early T cell marker CD27. In addition, iT_{SCM} cells that were obtained by co-culturing activated T cells that had been transduced with FOXM1ΔN or an empty vector (FOXM1ΔN or Empty) with OP9-hDLL1 cells were analyzed for the expression as a positive control (Table 1, and Figs. 5 and 6). These cells were also analyzed for the expression of FOXM1 (Fig. 7).

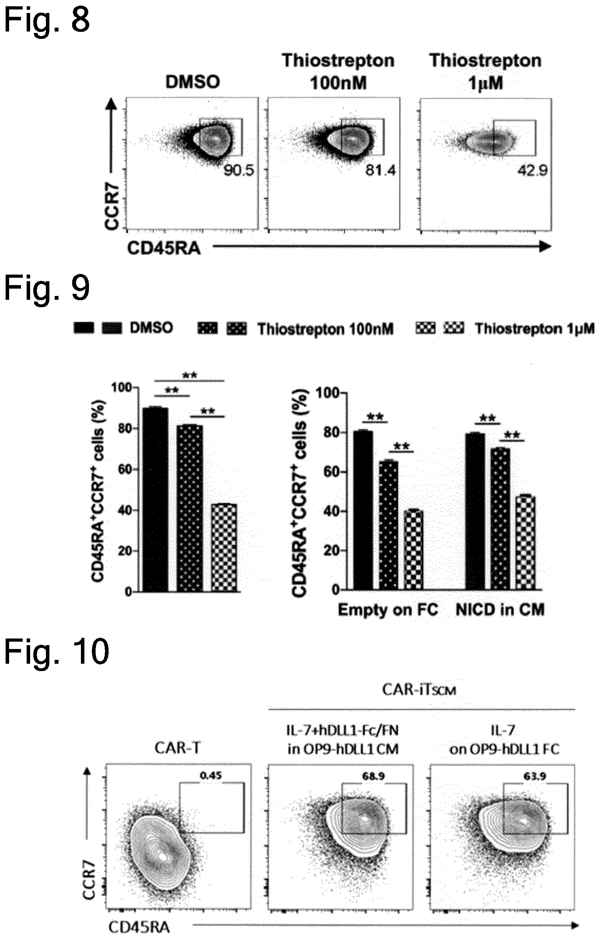

Moreover, activated T cells transduced with FOXM1ΔN or the empty vector were co-cultured with OP9-hDLL1 cells in the presence of a FOXM1 inhibitor Thiostrepton. The resulting cells were analyzed for the expression of CD45RA and CCR7 (Figs. 8, and 9).

Figs. 8 and 9 and Table 1 confirm that the combination of Notch signal activation resulting from the forced expression of FOXM1 and culture in the presence of OP9-hDLL1 derived-conditioned medium allowed the expression of CD45RA⁺ and CCR7⁺ as in co-culturing with OP9-hDLL1 cells (FC) and that the FOXM1-forcedly expressing cells also expressed the early T cell marker CD27 at a high level (CM).

Fig. 7 confirms that the transduction of T cells with FOXM1 markedly increased the expression of FOXM1, as compared with T cells only cultured in the presence of the conditioned medium derived from Notch ligand-expressing stromal cells (OP9-hDLL1 cells).

The results shown in Figs. 8 and 9 confirm that the inhibition of FOXM1 effect in the co-culture suppresses induction of CD45RA⁺CCR7⁺, and induction of FOXM1 in the culture in the presence of the conditioned medium also suppresses induction of CD45RA⁺CCR7⁺, indicating that FOXM1 is a downstream gene in Notch signaling and induces CD45RA⁺CCR7⁺.

### Results of the analysis of surface marker expression of stem cell memory-like T cells

Table 1 collectively shows the results of the analysis of surface marker expression on the stem cell memory-like T cells produced by combining Notch signal activation and culture in the presence of OP9-hDLL1-derived conditioned medium, in Examples 1 to 4.

### [Table 1]

**Table 1. Expression of various surface markers on T cells**

| Example | Method | CD45RA | CCR7 |
|---|---|---|---|
| 1 | OP9-hDLL1-derived conditioned medium (CM) | + | + |
| | Co-culture with OP9-hDLL1 cells (FC) | + | + |
| 2 | OP9 cell-derived conditioned medium (CM) | + | + |
| | TSt-4 cell-derived conditioned medium (CM) | + | + |
| 3 | OP9-hDLL1-derived conditioned medium, forced expression of NICD (NICD in CM) | + | + |
| | OP9-hDLL1-derived conditioned medium, empty vector (Empty in CM) | + | + |
| | Co-culture with OP9-hDLL1 cells, forced expression of NICD (NICD on FC) | + | + |
| | Co-culture with OP9-hDLL1 cells, empty vector (Empty on FC) | + | + |
| 4 | OP9-hDLL1-derived conditioned medium, forced expression of FOXM1ΔN (NICD in CM) | + | + |
| | OP9-hDLL1-derived conditioned medium, empty vector (Empty in CM) | + | + |
| | Co-culture with OP9-hDLL1 cells, forced expression of FOXM1ΔN (NICD on FC) | + | + |
| | Co-culture with OP9-hDLL1 cells, empty vector (Empty on FC) | + | + |

It can be confirmed that the combination of the culturing step in the presence of a conditioned medium derived from stromal cells and Notch signal activation resulting from the forced expression of NICD or FOXM1ΔN in activated T cells allowed the preparation of T cells expressing CD45RA⁺ and CCR7⁺.

The results shown in Figs. 3, 5 and 6 reveal that the number of CD45RA⁺CCR7⁺ cells obtained was greatly increased in the combination of culture in the presence of the conditioned medium and Notch signal activation, as compared with the combination of co-culture and Notch signal activation. In other words, it is suggested that Notch signal activation in the production method of the present invention can efficiently produce stem cell memory-like T cells, as compared with the co-culture system.

### Example 5: Method of producing stem cell memory-like T cells using OP9-hDLL1-derived conditioned medium/Notch signal activation (recombinant DLL1)

### 5-1. Preparation of activated anti-CD19 CAR-T cells

An anti-CD19 CAR overexpression retroviral vector consisting of anti-CD19 scFv (derived from FMC63), human CD8α hinge domain and a transmembrane domain, and human 4-1BB-CD3z intracellular signal motif was used to prepare CD8α⁺ T cells forcedly expressing anti-CD 19 CAR in a similar manner to the method of producing NICD-forcedly expressing activated CD8α⁺ T cells described in Example 3. Specifically, human CD8α⁺ T cells were activated in a similar manner to the Priming step described in Example 1. Twenty-four hours after the activation, the activated T cells were transduced with the anti-CD 19 CAR overexpression retroviral vector to achieve the forced expression of the anti-CD19 CAR. Six days after the activation of T cells, the cells were sorted for T cells forcedly expressing anti-CD 19 CAR as Venus⁺ cells. The anti-CD19 CAR-T cells were then re-activated with X-irradiated leukemia cells forcedly expressing human CD19 (K562-hCD19). In other words, a cell suspension comprising anti-CD19 CAR-T cells and K562-hCD19 cells (anti-CD19 CAR-T cells: 2.5 x 10⁵ cells/mL, K562-hCD19 cells: 0.625 × 10⁵ cells/mL) were seeded onto a U-bottom 96-well plate (200 µL/well). After four days of culture, the cells were subjected to the same activation treatment and cultured for additional 4 days. The cells were sorted for CD8α⁺ T cells forcedly expressing anti-CD19 CAR as CD8α⁺Venus⁺CD45RA⁻ cells to obtain activated CD8α⁺ T cells forcedly expressing anti-CD 19 CAR.

### 5-2. Preparation of hDLL1-Fc protein

The extracellular domain of human DLL1 cloned from human cDNA was amplified by PCR, and the amplified product was subcloned into a pcDNA3-hIgG1-Fc vector to produce a pcDNA3-hDLL1hIgG1-Fc vector. Expi293F cells (Thermo Fisher Scientific Inc.) were seeded onto a dish coated with 0.1% gelatin (10⁷ cells/26 mL/15 cm dish (Falcon, 353025)). Twenty-four hours later, the Expi293F cells were transfected with the pcDNA3-hDLL1-hIgG1-Fc vector using PEI MAX (Polysciences, 24765-1). Sixteen hours after the transfection, the cells were washed with Dulbecco's modified Eagle's medium (DMEM, NACALAI TESQUE, INC., 0845964) once, and the medium was replaced with a prepared serum-free medium (26 mL). The serum-free media was DMEM/F12 (NACALAI TESQUE, INC., 11581-15) supplemented with bovine serum albumin (BSA, NACALAI TESQUE, INC., 01863-48, 0.5%), penicillin-streptomycin (NACALAI TESQUE, INC., 26253-84, 1%), HEPES (NACALAI TESQUE, INC., 17557-94, 1 mM), sodium pyruvate (NACALAI TESQUE, INC., 06977-34, 1 mM), MEM Non-Essential Amino Acid Solution (NACALAI TESQUE, INC., 06344-56, 100-fold diluted), L-glutamine (NACALAI TESQUE, INC., 16948-04, 2 mM), ITS-X (Gibco, 51500-056, 100-fold diluted), and lipid concentrate (Chemically defined Lipid Concentrate, Gibco, 11905-031, 1000-fold diluted). Seventy-two hours after the medium replacement, the conditioned medium was harvested, filtered through a sterile filter (pore size: 0.22 µm), and stored at 4°C.

### 5-3. Preparation of recombinant DLL1-treated plates

Protein A (Sigma-Aldrich Co. LLC, P6031, 10 µg/mL) and fibronectin (FUJIFILM Wako Pure Chemical Corporation, 063-05591, 5 µg/mL) diluted with phosphate-buffered physiological saline (PBS) were adsorbed onto a non-treated 12-well plate (Falcon, 351143, 800 µL/well) at 4°C overnight. The wells were washed with PBS twice and blocked with a blocking buffer (HBSS/2% BSA) at 25°C for 45 minutes. The blocking buffer was removed by washing the wells with PBS twice, and then hDLL1-Fc (4 mL) was added to the protein A/fibronectin-coated wells. The plate was then incubated at 37°C in 5 vol.% CO₂ for 2 hours. The wells were washed with PBS twice before seeding cells.

### 5-4. Culture of activated anti-CD 19 CAR-T cells

The prepared activated CD8α⁺ T cells forcedly expressing anti-CD 19 CAR were seeded onto the prepared recombinant DLL1-treated plate at a density of 1 × 10⁵ cells/well and cultured for 11 days in the presence of OP9-hDLL1-derived conditioned medium (CM, 1 mL) supplemented with human IL-7 (Peprotech, 200-07, 10ng/mL) to obtain stem cell memory-like anti-CD19 CAR-T cells.

### 5-5. Analysis of surface marker expression

The obtained stem cell memory-like anti-CD 19 CAR-T cells were examined for reactivity to marker specific antibodies in a similar manner to Example 1. Cells cultured without recombinant DLL1 or the conditioned medium were also analyzed as a negative control, and cells co-cultured with feeder cells (FC) were also analyzed as a positive control. The results are shown in Fig. 10.

Fig. 10 confirms that the combination of Notch signal activation resulting from recombinant DLL1 and culture in the presence of OP9-hDLL1-derived conditioned medium allowed the preparation of T cells expressing the surface antigen markers CD45RA and CCR7 as in co-culturing with OP9-hDLL1 cells.

### Example 6: Analysis of active factors produced from stromal cells

### 6-1. Analysis of gene expression in OP9-hDLL1 cells by real-time PCR

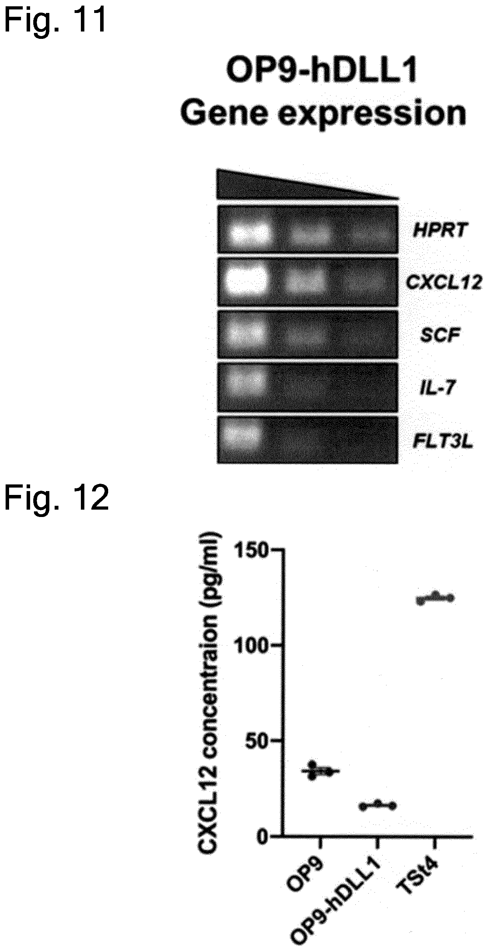

Total RNA was purified from 10⁵ OP9-hDLL1 cells using a ReliaPrep^{™} RNA Cell Miniprep System (Promega, Z6012). The total RNA obtained from OP9-hDLL1 was subjected to real-time PCR using PCT kit (High Capacity cDNA Reverse Transcription Kit, Applied Biosystems, 4368813) to obtain single-stranded cDNA. The obtained OP9-hDLL1 cDNA was serially diluted (1:1, 1:16, 1:256), and the diluted cDNA was used as a template to amplify genes of interest (HPRT, CXCL12, SCF, IL-7, and FLT3L) by an intercalator-based real-time PCR (CFX Connect (BioRad)) with SsoFast EvaGreen Supermix (BioRad, 172-5203). The obtained PCR amplified products of the genes of interest were applied to 1% agarose gel with a dye (Loading dye, TOYOBO, RE-DYE) for the visualization and subjected to electrophoresis. The results are shown in Fig. 11.

### Primers used

- Mouse HPRT
   Forward primer: CTTTGCTGACCTGCTGGATT (SEQ ID NO: 4)
   Reverse primer: TATGTCCCCCGTTGACTGAT (SEQ ID NO: 5)
- Mouse CXCL12
   Forward primer: GCCAACGTCAAGCATCTGAAA (SEQ ID NO: 6)
   Reverse primer TGTCTGTTGTTGTTCTTCAGCC (SEQ ID NO: 7)
- Mouse SCF
   Forward primer: GCGGGAATCCTGTGACTGAT (SEQ ID NO: 8)
   Reverse primer: CATCCCGGCGACATAGTTGA (SEQ ID NO: 9)
- Mouse IL-7
   Forward primer: ACATCATCTGAGTGCCACATT (SEQ ID NO: 10)
   Reverse primer: GGGCAATTACTATCAGTTCCTGT (SEQ ID NO: 11)
- Mouse FLT3L
   Forward primer: TGTTACTTCAGCCACAGTCCC (SEQ ID NO: 12)
   Reverse primer: GAAGATTGACGGCCACAGTGA (SEQ ID NO: 13)

### 6-2. Determination of CXCL12 in a conditioned medium derived from stromal cells

Conditioned media derived from OP9 cells, OP9-hDLL1 cells, and TSt-4 cells (OP9 CM, OP9-hDLL1 CM, and TSt-4 CM) were prepared. Cells of these cell lines were individually seeded onto a dish for cell culture (10 cm, Falcon, 353003) at 1.5 × 10⁵ cells/dish and grown in α-MEM medium (Gibco, 11900-024, 1.5 mL) supplemented with fetal bovine serum (FBS, 20%, Sigma-Aldrich Co. LLC, 172012) and penicillin-streptomycin (1%, NACALAI TESQUE, INC., 26253-84). The cells were grown under the conditions at 37°C in 5 vol.% CO₂. When the cells reached 100% confluence, the media (OP9 CM, OP9-hDLL1 CM, and TSt-4 CM) were harvested, filtered through a sterile filter (pore size: 0.45 µm), and stored at 4°C. CXCL12 contained in the harvested conditioned media was quantified by enzyme-linked immunosorbent assay (ELISA) (Invitrogen, EMCXCL12). The results are shown in Fig. 12.

The results shown in Fig. 11 indicate that, of candidate active factors examined, CXCL12 was expressed at an especially high level. The results shown in Fig. 12 confirm that CXCL12 was actually present in conditioned medium derived from OP9-hDLL1 and that CXCL12 was also present in the conditioned media derived from OP9 cells and TSt4 cells.

### Example 7: Method of producing stem cell memory-like anti-CD 19 CAR-T cells using CXCL12/IGF-I/Notch signal activation

### 7-1. Culture of activated T cells

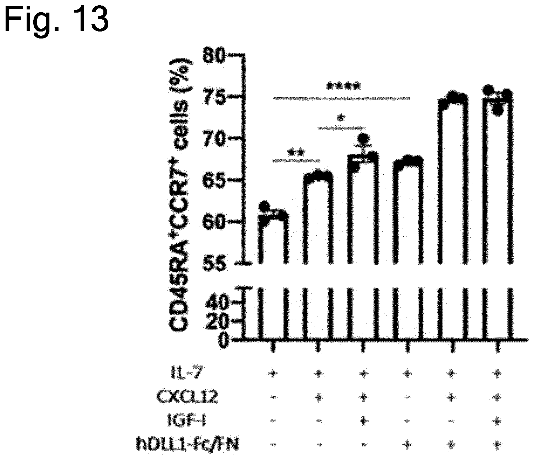

Activated CD8α⁺ T cells forcedly expressing anti-CD19 CAR thus prepared were seeded onto a recombinant DLL1-treated plate prepared in a similar manner to Example 5 (hDLL1-Fc/FN) or an untreated plate at a density of 1 × 10⁵ cells/well. The cells were cultured for 11 days in α-MEM medium (Gibco, 11900-24, 1 mL) supplemented with human IL-7 (Peprotech, 200-07, 10 ng/mL), fetal bovine serum (FBS, 20%, Sigma-Aldrich Co. LLC, 172012), and penicillin-streptomycin (1%, NACALAI TESQUE, INC., 26253-84) and further a combination of human CXCL12 (Peprotech, 300-28A, 100 ng/mL) and human IGF-I (Peprotech, 100-11, 50 ng/mL) shown in Fig. 13 to obtain stem cell memory-like anti-CD 19 CAR-T cells.

### 7-2. Analysis of surface marker expression

The obtained stem cell memory-like anti-CD 19 CAR-T cells were examined for reactivity to marker specific antibodies in a similar manner to Example 1. The results are shown in Fig. 13.

Fig. 13 confirms that, instead of a conditioned medium derived from stromal cells, the medium supplemented with CXCL12 can be also used to induce stem cell memory-like T cells. In addition, it was also confirmed that the induction of stem cell memory-like T cells was enhanced by the addition of IGF-I and the treatment with recombinant DLL1.

### Example 8: Anti-tumor effect of stem cell memory-like anti-CD 19 CAR-T cells produced using CXCL12/IGF-I/Notch signal activation

### 8-1. Evaluation of anti-tumor effect of anti-CD19 CAR-T cells in human leukemia model mice

NALM6 cells were suspended in PBS at a density of 5 x 10⁶ cells/mL, and 200 µL of the cell suspension was injected to NSG mice (NOD-SCID IL-2Rγ^{null} mouse) via tail vein. Seven days after the transplantation of NALM6 cells, stem cell memory-like anti-CD19 CAR-T cells prepared in a similar manner to Example 7 (FF CAR-iT_{SCM}) or anti-CD 19 CAR-T cells that had not been induced to be stem cell memory-like (CAR-T) were suspended in PBS at a density of 2.5 × 10⁶ cells/mL, and 200 µL of the cell suspension was injected to NSG mice via tail vein. Fourteen days after the transplantation of anti-CD19 CAR-T cells, 40 µL of blood was taken from each of the NSG mice and hemolyzed with a buffer (ACK Lysis buffer) to obtain a cell suspension.

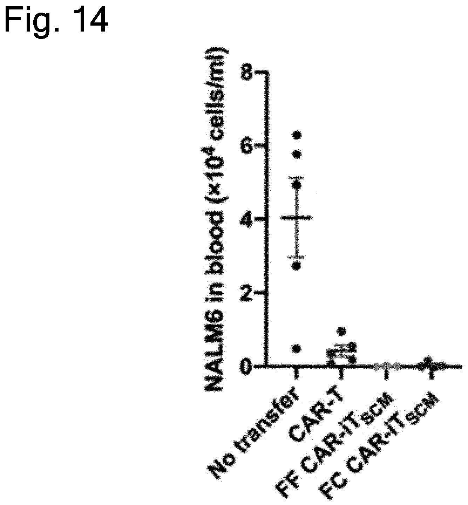

The expression of surface antigen markers in the obtained cell suspension were determined by flow cytometric analysis to count the number of FVD⁻mCD45⁻hCD19⁺ NALM6 cancer cells. This study also included a group of mice to which CAR-T cells had not been administered and a group of mice to which stem cell memory-like anti-CD 19 CAR-T cells produced by co-culture with feeder cells (FC CAR-iT_{SCM}) had been administered, and these groups were also evaluated. The results are shown in Fig. 14.

### Antibodies and reagents used

BV421 anti-mouse CD45 Antibody (BioLegend, 103133) (Clone: 30-F11)
PE anti-human CD19 Antibody (BioLegend, 302208) (Clone: 4G7)
Fixable Viability Dye eFluor^{™} 780 (eBiosciences, 65-0865-18)

Fig. 14 confirms that stem cell memory-like anti-CD19 CAR-T cells produced using CXCL12/IGF-I/Notch signal activation demonstrated potent antitumor activity.

### INDUSTRIAL APPLICABILITY

The present invention solves the problem in T-cell transfer therapy and the like, which is T-cell exhaustion, and can enhance T cell activity. Therefore, the present invention is expected to be applied to adoptive T cell therapy and CAR-T cell therapy.

## Claims

1. A method of producing T cells having cell surface markers of CD45RA⁺ and CCR7⁺, wherein the method comprises a step of culturing source T cells in the presence of (a) a conditioned medium derived from stromal cells or (b) CXCL12.

2. The method according to claim 1, further comprising a step of activating the source T cells before the culturing step.

3. The method according to claim 1 or 2, wherein the stromal cell is any one or more selected from the group consisting of OP9 cell, TSt-4 cell, and these cells engineered to express a Notch ligand.

4. The method according to any one of claims 1 to 3, wherein the stromal cell is OP9-DLL1 cell.

5. The method according to any one of claims 1 to 4, further comprising activating Notch signal before or during the culturing step.

6. The method according to claim 5, wherein the activation of Notch signal is performed using any one or more Notch ligands selected from the group consisting of DLL4, DLL1, JAG1, JAG2, and a recombinant thereof.

7. The method according to claim 5 or 6, wherein the activation of Notch signal is performed using a recombinant DLL1.

8. The method according to any one of claims 5 to 7, wherein the activation of Notch signal is performed using forced expression of FOXM1 or NICD.

9. The method according to any one of claims 1 to 8, wherein the T cell having the cell surface markers of CD45RA⁺ and CCR7⁺ has cell surface markers of CD45RO⁻ and CD62L⁺.

10. The method according to any one of claims 1 to 9, the source T cell is a CAR-T cell.

11. The method according to any one of claims 1 to 10, wherein IGF-I is added in the step of culturing in the presence of CXCL12.

12. AT cell produced by the method according to any one of claims 1 to 11.

13. A cellular medicine comprising the T cells according to claim 12.

14. The cellular medicine according to claim 13, for the treatment of one or more selected from the group consisting of a cancer, an infection, an autoimmune disease, and an allergy.


SEQUENCE LISTING

<110>  Daiichi Sankyo Company, Limited
       Keio University

<120>  METHOD FOR PREPARING T CELL HAVING CELL SURFACE MARKERS OF CD45RA+
and CCR7+

<130>  FA1511-20195

<150>  JP2019-195206
<151>  2019-10-28

<160>  13

<170>  PatentIn version 3.5

<210>  1
<211>  2412
<212>  DNA
<213>  Homo sapiens

<400>  1
atggtgctgc tgtcacgcaa aaggaggaga caacacggac agctgtggtt tcccgaaggt      60

tttaaggtga gtgaagcctc taagaagaaa cggcgcgagc cactgggcga agattccgtt     120

ggacttaagc ctttgaagaa tgcctcagat ggagcgctta tggacgacaa ccagaacgag     180

tggggagacg aggaccttga gacgaagaag ttccgattcg aagaaccggt ggtcctgcca     240

gacctcgatg accagacaga ccaccgtcag tggacacagc agcacctgga cgcagctgat     300

ctccggatgt cagccatggc ccccactcca ccccagggcg aagtggatgc cgattgcatg     360

gatgtcaatg tgaggggtcc cgatggtttc acacctctga tgattgcctc ttgcagtgga     420

gggggcctgg agacaggcaa ttccgaggag gaggaagatg caccggccgt catctcggat     480

ttcatctacc aaggggcatc cctccacaat cagaccgata gaacaggcga gactgcgctg     540

cacctggcgg ctcggtattc ccgctcagat gccgccaaaa ggctattaga agcaagcgct     600

gacgccaata tccaagacaa catgggcaga actcccctgc atgccgcggt gtctgccgat     660

gcgcagggtg tgttccagat tctgattagg aatagagcga ctgatctcga tgcaagaatg     720

cacgatggta ctacccctct catcctggca gccagacttg ccgttgaagg gatgttggag     780

gacttaatca actcacatgc ggacgtgaat gctgtggacg atctcggcaa aagtgcactc     840

cattgggccg ccgctgtgaa caacgtcgat gccgcagtgg tgctcttgaa gaacggagcc     900

aataaggaca tgcagaataa tcgggaggag actccattgt ttctggcagc tagggagggg     960

tcctatgaga cagccaaagt cctgcttgat cacttcgcta atcgagacat cacagaccac    1020

atggatcgcc tgccgagaga catagctcaa gagcgaatgc accacgacat tgtacggttg    1080

ctggacgagt acaacttggt gcgctctcct cagttgcatg gcgcacctct gggcggtact    1140

ccaaccctga gccccccact gtgtagcccc aatggctacc tgggcagcct taagccaggc    1200

gtccagggca aaaaggtcag gaagccctcc agcaaaggcc tggcatgtgg gagtaaggag 1260

gccaaagatc tcaaagcccg acggaaaaaa tcccaggacg gcaaaggatg cctcttggac 1320

tcctcaggga tgctgagccc cgttgactcc ctcgagtccc ctcatgggta ccttagcgac 1380

gttgcttctc cacccctatt gccttcaccc tttcagcaaa gcccctctgt tcctctgaat 1440

cacctacccg gcatgccaga tacccatctg ggaataggcc acctcaatgt ggctgccaag 1500

ccagaaatgg ctgctctggg aggggaggga cgcttggcct ttgaaaccgg acctcctcgt 1560

ctaagtcatc ttcccgtggc atctggcaca agtacggtgt taggatcttc gtcgggcgga 1620

gcacttaact tcaccgttgg gggcagcacc agcttgaatg ggcaatgtga gtggctgtcc 1680

agactccagt ctggtatggt acccaaccag tataaccctt tacggggtag tgttgctcct 1740

ggccccctgt ctacgcaggc cccaagtctc cagcacggaa tggtaggtcc tctgcattct 1800

agcttagctg cctccgctct cagccagatg atgagctatc aggggctgcc ttcaaccagg 1860

ctcgctaccc aaccccatct cgtccaaacc cagcaagtgc aaccacagaa cttgcagatg 1920

cagcagcaaa accttcagcc agccaacatt cagcagcagc agagcctgca gcctccgcct 1980

ccaccaccgc aaccacacct gggggtgtct tcagccgcgt ccggacatct ggggcgcagt 2040

ttcctaagcg gggaacctag tcaggctgac gtccaaccac ttggtccgag ctcgttagcc 2100

gtgcacacca tactgcctca ggaatctccc gctctaccta ctagcctgcc gtcctccctg 2160

gtaccccccg tgacagcagc ccaattctta acgccaccgt cccagcattc ctactcctca 2220

cctgtggaca acacccctag tcatcagctg caggttcccg aacacccgtt tcttacaccc 2280

tcacccgaat ctcctgatca gtggagcagc tcctcgccac actcaaacgt aagtgactgg 2340

tctgaggggg tcagcagtcc accaacctct atgcagtcac agatcgctcg tattcccgag 2400

gcatttaagt ga 2412

<210> 2
<211> 1560
<212> DNA
<213> Homo sapiens

<400> 2
atgaactctg tgtctgagcg gccaccctac tcttacatgg ccatgataca attcgccatc 60

aacagcactg agaggaagcg catgactttg aaagacatct atacgtggat tgaggaccac 120

tttccctact ttaagcacat tgccaagcca ggctggaaga actccatccg ccacaacctt 180

tccctgcacg acatgtttgt ccgggagacg tctgccaatg gcaaggtctc cttctggacc 240

attcacccca gtgccaaccg ctacttgaca ttggaccagg tgtttaagca gcagaaacga 300

ccgaatccag agctccgccg gaacatgacc atcaaaaccg aactccccct gggcgcacgg 360

cggaagatga agccactgct accacgggtc agctcatacc tggtacctat ccagttcccg 420

gtgaaccagt cactggtgtt gcagccctcg gtgaaggtgc cattgcccct ggcggcttcc 480

ctcatgagct cagagcttgc ccgccatagc aagcgagtcc gcattgcccc caaggtgctg 540

ctagctgagg aggggatagc tcctctttct tctgcaggac cagggaaaga ggagaaactc 600

ctgtttggag aagggttttc tcctttgctt ccagttcaga ctatcaagga ggaagaaatc 660

cagcctgggg aggaaatgcc acacttagcg agacccatca aagtggagag ccctcccttg 720

gaagagtggc cctccccggc cccatctttc aaagaggaat catctcactc ctgggaggat 780

tcgtcccaat ctcccacccc aagacccaag aagtcctaca gtgggcttag gtccccaacc 840

cggtgtgtct cggaaatgct tgtgattcaa cacagggaga ggagggagag gagccggtct 900

cggaggaaac agcatctact gcctccctgt gtggatgagc cggagctgct cttctcagag 960

gggcccagta cttcccgctg ggccgcagag ctcccgttcc cagcagactc ctctgaccct 1020

gcctcccagc tcagctactc ccaggaagtg ggaggacctt ttaagacacc cattaaggaa 1080

acgctgccca tctcctccac cccgagcaaa tctgtcctcc ccagaacccc tgaatcctgg 1140

aggctcacgc ccccagccaa agtaggggga ctggatttca gcccagtaca aacctcccag 1200

ggtgcctctg accccttgcc tgaccccctg gggctgatgg atctcagcac cactcccttg 1260

caaagtgctc ccccccttga atcaccgcaa aggctcctca gttcagaacc cttagacctc 1320

atctccgtcc cctttggcaa ctcttctccc tcagatatag acgtccccaa gccaggctcc 1380

ccggagccac aggtttctgg ccttgcagcc aatcgttctc tgacagaagg cctggtcctg 1440

gacacaatga atgacagcct cagcaagatc ctgctggaca tcagctttcc tggcctggac 1500

gaggacccac tgggccctga caacatcaac tggtcccagt ttattcctga gctacagtag 1560

<210> 3
<211> 1479
<212> DNA
<213> Artificial Sequence

<220>
<223> Anti-CD19 scFv (derived from FMC63), human CD8 alpha hinge domain
and transmembrane domain, and human CD28-CD3z intracellular
signal motif

<400> 3
atgcttctcc tggtgacaag ccttctgctc tgtgagttac cacacccagc attcctcctg 60

atcccagaca tccagatgac acagactaca tcctccctgt ctgcctctct gggagacaga 120

gtcaccatca gttgcagggc aagtcaggac attagtaaat atttaaattg gtatcagcag 180

aaaccagatg gaactgttaa actcctgatc taccatacat caagattaca ctcaggagtc 240

ccatcaaggt tcagtggcag tgggtctgga acagattatt ctctcaccat tagcaacctg 300

gagcaagaag atattgccac ttacttttgc caacagggta atacgcttcc gtacacgttc 360

ggagggggga ctaagttgga aataacaggc tccacctctg gatccggcaa gcccggatct 420

ggcgagggaa gcaccaaggg cgaggtgaaa ctgcaggagt caggacctgg cctggtggcg 480

ccctcacaga gcctgtccgt cacatgcact gtctcagggg tctcattacc cgactatggt 540

gtaagctgga ttcgccagcc tccacgaaag ggtctggagt ggctgggagt aatatggggt 600

agtgaaacca catactataa ttcagctctc aaatccagac tgaccatcat caaggacaac 660

tccaagagcc aagttttctt aaaaatgaac agtctgcaaa ctgatgacac agccatttac 720

tactgtgcca aacattatta ctacggtggt agctatgcta tggactactg gggtcaagga 780

acctcagtca ccgtctcctc agcggccgca accacaaccc cagcacctcg cccaccaaca 840

ccagcaccaa ccatcgcatc ccagccactg tctctgcggc ccgaggcctg taggcctgca 900

gcaggaggag cagtgcacac caggggcctg gactttgcct gcgatatcta tatctgggca 960

ccactggcag gaacatgtgg cgtgctgctg ctgtccctgg tcatcaccct gtattgcagg 1020

agtaagagga gcaggctcct gcacagtgac tacatgaaca tgactccccg ccgccccggg 1080

cccacccgca agcattacca gccctatgcc ccaccacgcg acttcgcagc ctatcgctcc 1140

agagtgaagt tcagcaggag cgcagacgcc cccgcgtacc agcagggcca gaaccagctc 1200

tataacgagc tcaatctagg acgaagagag gagtacgatg ttttggacaa gagacgtggc 1260

cgggaccctg agatgggggg aaagccgaga aggaagaacc ctcaggaagg cctgtacaat 1320

gaactgcaga aagataagat ggcggaggcc tacagtgaga ttgggatgaa aggcgagcgc 1380

cggaggggca aggggcacga tggcctttac cagggtctca gtacagccac caaggacacc 1440

tacgacgccc ttcacatgca ggccctgccc cctcgctaa 1479

<210> 4
<211> 20
<212> DNA
<213> Artificial Sequence

<220>
<223> forward primer for mouse HPRT

<400> 4
ctttgctgac ctgctggatt 20

<210> 5
<211> 20
<212> DNA
<213> Artificial Sequence

<220>
<223> reverse primer for mouse HPRT

<400> 5
tatgtccccc gttgactgat 20

<210> 6

<211>  21
<212>  DNA
<213>  Artificial Sequence

<220>
<223>  forward primer for mouse CXCL12

<400>  6
gccaacgtca agcatctgaa a                                                21


<210>  7
<211>  22
<212>  DNA
<213>  Artificial Sequence

<220>
<223>  reverse primer for mouse CXCL12

<400>  7
tgtctgttgt tgttcttcag cc                                               22


<210>  8
<211>  20
<212>  DNA
<213>  Artificial Sequence

<220>
<223>  forward primer for mouse SCF

<400>  8
gcgggaatcc tgtgactgat                                                  20


<210>  9
<211>  20
<212>  DNA
<213>  Artificial Sequence

<220>
<223>  reverse primer for mouse SCF

<400>  9
catcccggcg acatagttga                                                  20


<210>  10
<211>  21
<212>  DNA
<213>  Artificial Sequence

<220>
<223>  forward primer for mouse IL-7

<400>  10
acatcatctg agtgccacat t                                                21


<210>  11
<211>  23
<212>  DNA
<213>  Artificial Sequence

<220>
<223>  reverse primer for mouse IL-7

<400>  11
gggcaattac tatcagttcc tgt                                              23


<210>  12
<211>  21
<212>  DNA
<213>  Artificial Sequence

<220>
<223>  forward primer for mouse FLT3L

<400>  12
tgttacttca gccacagtcc c                                                21


<210>  13
<211>  21
<212>  DNA
<213>  Artificial Sequence

<220>
<223>  reverse primer for mouse FLT3L

<400>  13
gaagattgac ggccacagtg a                                                21